# DEMANDE DE BREVET EUROPEEN

(11) **EP 2 609 942 A1**
(43) Date de publication de la demande: **03.07.2013**
(21) Numéro de dépôt: 12306517.9
(22) Date de dépôt: 05.12.2012
(51) Int. Cl.: A61M 1/06

(54) **Dispositif anti retour de fluides incorporé dans un ensemble d'expression de lait maternel**

(30) Priorité: 29.12.2011 FR 1162510
(71) Demandeur: LA DIFFUSION TECHNIQUE FRANCAISE, 42000 Saint Etienne (FR)
(72) Inventeur: Chantrel, Gilles, 42100 Saint-Etienne (FR); Massardier, Michel, 42000 Saint-Etienne (FR)
(74) Mandataire: Dupuis, François

(57) **Abrégé**

Le dispositif anti-retour de fluides incorporé dans un ensemble d'expression de lait maternel comprenant un biberon (1) et une téterelle (2) présentant en une partie d'extrémité un bout de sein ; la dite téterelle se caractérise en ce que elle est connectée à un clapet anti-retour, et à un moyen support (4) tubulaire entourant ledit clapet anti-retour (3) et débordant par rapport à l'extrémité dudit clapet anti-retour de manière à assurer sa protection totale et le protéger de tout contact involontaire des doigts de l'utilisateur.

## Description

L'invention se rattache au secteur technique des ensembles d'expression de lait maternel qui sont conçus avec une téterelle incluant une partie bout de sein et s'adaptant sur le corps d'un biberon.

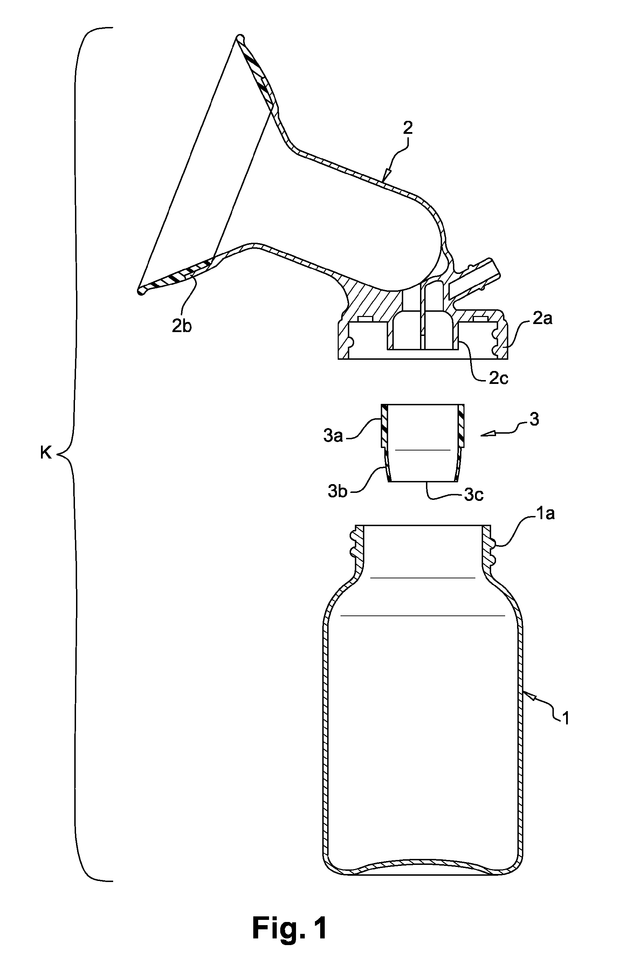

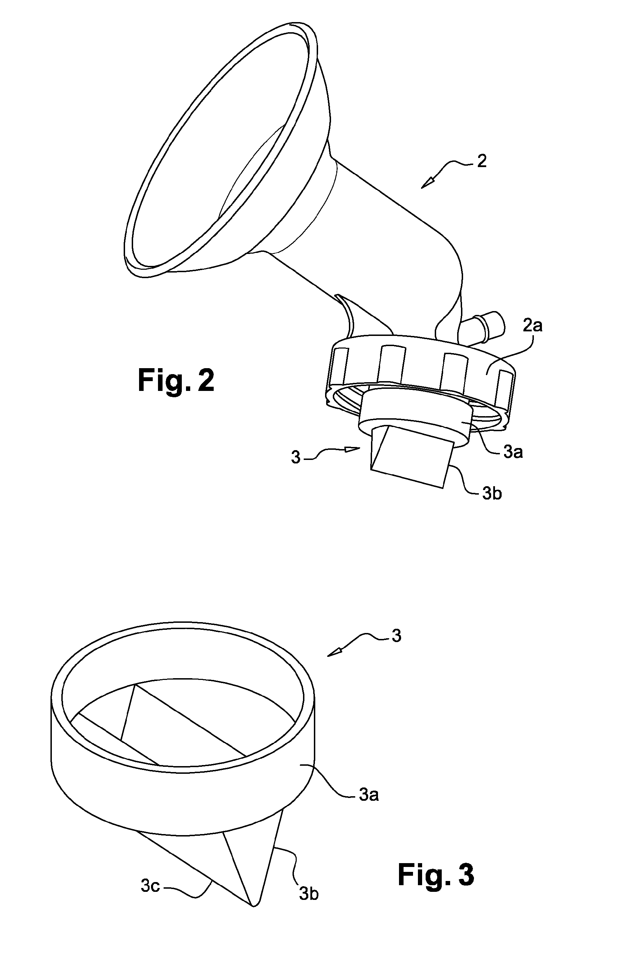

On a illustré selon les figures 1 à 3 l'art antérieur connu à partir notamment d'un exemple de fabrication et de conception d'un kit d'expression de lait maternel, et qui met en oeuvre les dispositions connues et reproduites également par la concurrence. L'ensemble d'expression de lait maternel (K) dénommé couramment dans les milieux professionnels par les termes "kit d'expression" comprend ainsi un biberon (1) et une téterelle (2) qui vient se visser par sa base (2a) sur la partie filetée (1a) du col du biberon. La téterelle (2) comprend également une partie d'extrémité dite "bout de sein" (2b) qui vient se positionner sur le sein de la personne allaitant. La téterelle est agencée dans le volume intérieur de la base (2a), avec un manchon cylindrique (2c) qui réduit le passage de circulation et de transfert du lait dans le biberon. Ce manchon est susceptible de recevoir un clapet anti-retour (3) qui est réalisé avec une base en forme de bague cylindrique (3a) prolongée par deux lèvres (3b) définissant entre elles une ouverture (3c) de passage du lait. Ce clapet anti-retour est réalisé en un matériau élastomère ayant une certaine capacité de déformation et pouvant être monté sur le manchon (2c) pour assurer sa fixation et son positionnement. Les deux lèvres (3b) en regard l'une de l'autre constituent également un moyen de préhension par un opérateur lorsqu'il y a lieu de procéder au nettoyage du clapet anti-retour. Les dites lèvres, comme représentées aux figures 2 et 3, sont inclinées l'une par rapport à l'autre, vers le bas, et permettent en formant buse d'écouler le lait dans le biberon.

On connaît par le brevet US 2007/0179439 la mise en oeuvre d'une pièce (70) dénommée diaphragme de configuration circulaire qui est logée, centrée et protégée par une chemise cylindrique (75).

On connaît également par le brevet US 2005/0154349 la mise en oeuvre d'un clapet anti-retour (2800) venant en débordement du cylindre l'entourant. Cette disposition est dans la même logique que l'Art Antérieur illustré figures 1 à 3 avec la possibilité pour l'utilisateur de saisir ledit clapet par les mains et ainsi, à la longue, de le détériorer.

La problématique réside dans le fait que ce clapet anti-retour (3) n'est nullement protégé en soi de l'environnement extérieur et surtout qu'il constitue en lui-même par la saisie des lèvres par les doigts de l'opérateur le moyen de préhension, de mise en place ou d'enlèvement par rapport à la téterelle. Les conditions d'hygiène sont donc très défavorables, sans compter en plus sur la déformation des dites lèvres par la multiplicité d'opérations de saisie pour nettoyage. On observe également que les différentes manipulations, à répétition, de mise en place du clapet anti-retour sur le manchon (2c) à l'intérieur de la base de la téterelle entraînent une déformation et un élargissement de la base du clapet anti-retour avec alors des risques d'échappement du clapet anti-retour dans le biberon.

La démarche du Demandeur a donc été de rechercher une solution qui permette une amélioration de l'hygiène quant à la manipulation de la téterelle, du nettoyage du clapet anti-retour, et qui évite aussi les risques de détérioration à la longue de la partie clapet s'adaptant sur le manchon récepteur prévu sur la téterelle.

La démarche du Demandeur a aussi été de rechercher une solution qui évite tout contact de l'opérateur ou de l'utilisateur directement avec le clapet anti-retour et notamment lors des phases de nettoyage.

La solution apportée par le Demandeur répond à ces objectifs d'une manière simple et pratique.

Selon une première caractéristique de l'invention, le dispositif anti-retour de fluides incorporé dans un ensemble d'expression de lait maternel comprenant un biberon et une téterelle présentant en une partie d'extrémité un bout de sein, la dite téterelle étant agencée pour la réception d'un clapet anti-retour, est remarquable en ce que la dite téterelle est agencée pour la réception d'un clapet anti-retour, et d'un moyen support tubulaire entourant le dit clapet anti-retour et débordant par rapport à l'extrémité dudit clapet anti-retour de manière à assurer sa protection totale et le protéger de tout contact involontaire des doigts de l'utilisateur.

Selon une autre caractéristique de l'invention, le dispositif anti-retour de fluides incorporé dans un ensemble d'expression de lait maternel, la dite téterelle étant agencée dans le volume intérieur de sa base avec un manchon cylindrique, est remarquable en ce que le moyen de protection forme support et est agencé intérieurement pour recevoir et maintenir un clapet anti-retour, le dit moyen support étant susceptible d'être positionné par rapport à la partie manchon formée dans la partie intérieure de la base constitutive du bout de sein, le dit moyen support étant de grande hauteur et assurant la protection du clapet anti-retour avec une hauteur de débordement par rapport à l'extrémité inférieure du clapet anti-retour comprise entre un millimètre et plusieurs centimètres afin d'empêcher le contact avec les doigts de l'utilisateur, et constituant le moyen de préhension et de manipulation par l'utilisateur et débouchant dans le dit biberon.

Selon une autre caractéristique de l'invention, le dispositif anti-retour de fluides incorporé dans un ensemble d'expression de lait maternel, la dite téterelle étant agencée dans le volume intérieur de sa base avec un manchon cylindrique, est remarquable en ce que le dit moyen support comprend deux constituants assemblés entre eux et enserrant un clapet anti-retour, le premier constituant étant positionné sur le dit manchon, et l'autre constituant assurant une double fonction d'une part de protection du clapet anti-retour et d'autre part constituant le moyen exclusif de préhension et de manipulation par l'utilisateur, le dit constituant débouchant dans le biberon.

Selon une autre caractéristique, la téterelle est agencée directement par moulage avec le moyen de protection du clapet anti-retour

Ces caractéristiques et d'autres encore ressortiront bien de la suite de la description.

Pour fixer l'objet de l'invention illustré d'une manière non limitative aux figures des dessins où :
La figure 1 est une vue éclatée avant montage d'un kit d'expression de lait maternel selon l'art antérieur en illustrant la partie téterelle, le biberon et le clapet anti-retour,
La figure 2 est une vue à grande échelle selon la figure 1 illustrant le montage du clapet anti-retour sur la partie réceptrice correspondante de la téterelle,
La figure 3 est une vue en perspective du clapet anti-retour selon la figure 1 et 2 de l'art antérieur,
La figure 4 est une vue en coupe illustrant le moyen support réalisé dans une variante de mise en oeuvre et réceptionnant le clapet anti-retour
La figure 5 est une vue de profil illustrant un kit d'expression de lait maternel mettant en oeuvre le dispositif anti-retour selon l'invention,
La figure 6 est une vue du kit d'expression de lait maternel selon la figure 4 en vue éclatée avant montage de ses composants,
La figure 7 est une vue à grande échelle illustrant en coupe le dispositif anti-retour selon l'invention,
La figure 8 est une vue à grande échelle, partielle et en perspective, en coupe, du dispositif selon la figure 7,
La figure 9 est une vue en perspective à grande échelle du dispositif anti-retour avant montage de ses composants,
La figure 10 est une vue en perspective illustrant le montage du dispositif anti-retour sur la téterelle.
La figure 11 est une vue en variante selon laquelle la téterelle est agencée directement lors de sa fabrication avec le moyen de protection du clapet anti-retour.
La figure 12 est une vue à caractère schématique d'une variante de réalisation, le moyen de protection du clapet anti-retour recevant en extrémité un moyen ajouré empêchant la pénétration de doigt de l'utilisateur.

Afin de rendre plus concret l'objet de l'invention, on le décrit maintenant d'une manière non limitative illustré aux figures des dessins.

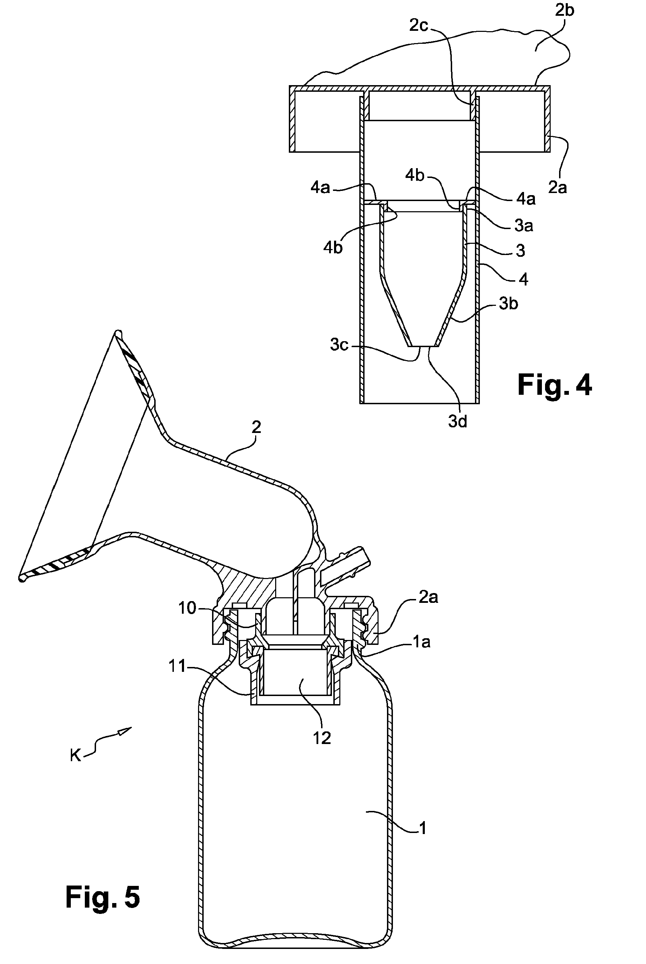

En se référant aux figures 4 à 9, le kit d'expression de lait maternel est référencé par (K) et comprend comme précédemment un biberon (1), une téterelle (2). Le biberon comprend un col (1a) sur lequel vient s'ajuster par vissage la base cylindrique (2a) de la téterelle. La configuration de celle-ci et notamment de sa partie "bout de sein" est établie de toute manière appropriée. La base (2a) comprend intérieurement une partie cylindrique ou conduit débouchant dans la partie supérieure de la téterelle et permettant ainsi le transfert du lait collecté dans le biberon.

Selon l'invention, la dite téterelle est agencée pour la réception d'un clapet anti-retour, et d'un moyen tubulaire entourant le dit clapet anti-retour de manière à assurer sa protection totale et en débordement par rapport à l'extrémité dudit clapet anti-retour pour assurer une fonction d'interdiction du passage des doigts de l'utilisateur. En outre, ledit moyen tubulaire constitue en lui-même un moyen de préhension et de manipulation. Différentes variantes de mise en oeuvre de l'invention sont ainsi décrites.

On a représenté figure 1 une première variante du dispositif anti-retour et de fixation du clapet anti-retour (3). Le dispositif comprend au moins un moyen support (4) de configuration tubulaire agencé intérieurement pour recevoir et maintenir le clapet anti-retour (3), le dit moyen support étant susceptible d'être positionné par rapport à la partie manchon (2c) formée dans la partie intérieure de la base (2a) constitutive du bout de sein (2b). Le dit moyen support (4) est de grande hauteur et assure la protection du clapet anti-retour, et constituant le moyen de préhension et de manipulation par l'utilisateur et débouchant dans le dit biberon. Le moyen support (4) présente ainsi intérieurement une collerette (4a) configurée en forme de 'L'avec une portée cylindrique (4b) sur laquelle vient se positionner une couronne (3a) cylindrique formée sur la base du clapet anti-retour (3). Ce dernier présente des lèvres (3b) qui sont convergentes pour définir un espace d'ouverture (3c) du passage du lait. Le moyen support ainsi réalisé est monobloc et est obtenu par moulage. Dans sa partie supérieure, il vient se positionner sur la partie manchon (2c) formée dans la base (2a) du bout de sein. La hauteur du moyen support (4) est telle qu'elle déborde par rapport à l'extrémité (3d) du clapet anti-retour. Ledit moyen (4) déborde de ladite extrémité entre un millimètre et plusieurs centimètres et avantageusement de l'ordre de 3 centimètres. Le moyen support (4) présente un diamètre (d) d'ouverture à son extrémité comprise entre 0,5 et 3 centimètre(s).

On a illustré aux figures suivantes une autre mise en oeuvre du dispositif anti-retour.

Le moyen support du clapet anti-retour est ainsi réalisé à partir d'un assemblage et comprend deux constituants (10) et (11) qui sont susceptibles de s'assembler entre eux de manière fixe ou démontable et enserrant un clapet anti-retour (12), le premier constituant (10), étant susceptible de se positionner sur le manchon intérieur (2c) de la base (2a) de la téterelle tandis que l'autre constituant (11) assure une double fonction, d'une part de protection du clapet anti-retour (12) et d'autre part constituant le moyen exclusif de préhension et de manipulation par l'utilisateur lors d'opérations d'entretien et de nettoyage du dit clapet anti-retour, le dit constituant (11) débouchant dans le biberon.

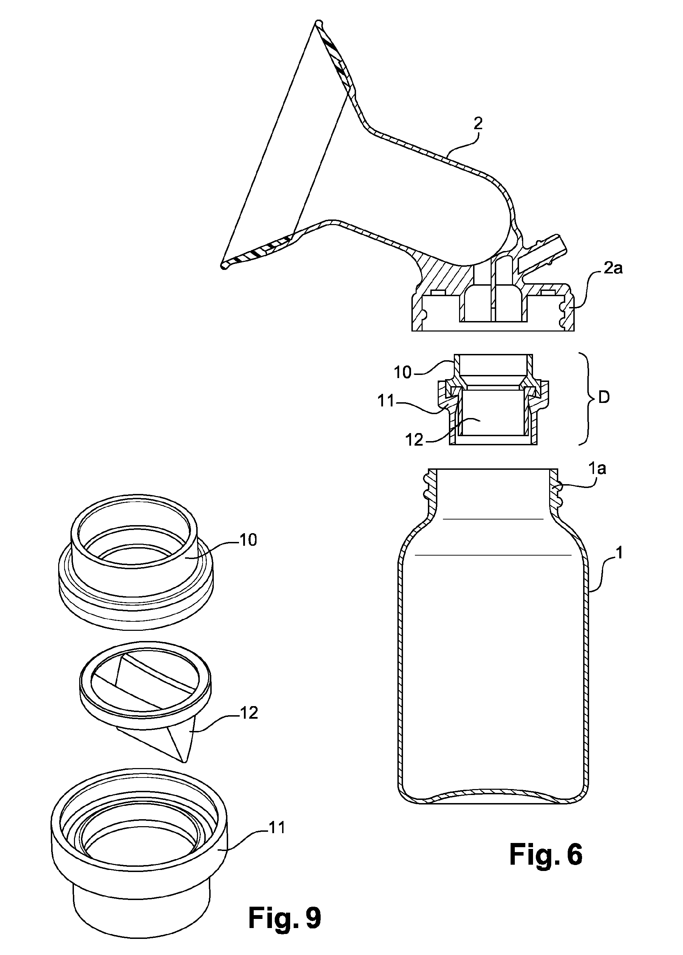

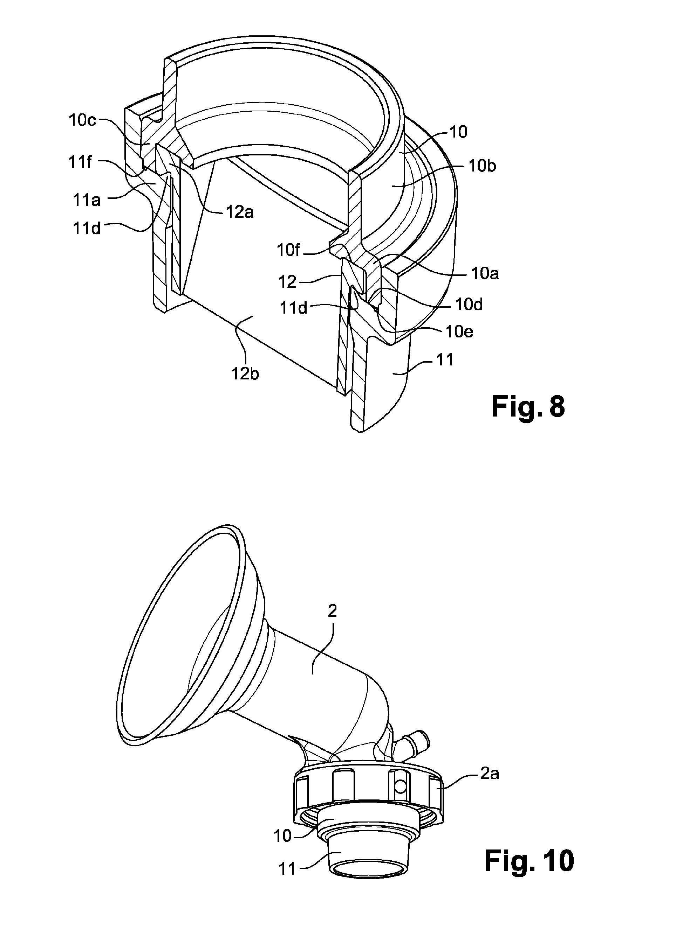

Le premier constituant (10) comprend une base centrale (10a) de configuration circulaire qui est prolongée d'une côté par une portée cylindrique (10b), qui est susceptible de venir s'emmancher sur le manchon (2c) précité, et de l'autre par une portée cylindrique (10c) de plus grand diamètre. Cette dernière présente en bout sur sa face avant (10d) un prolongement en saillie (10e) établi sur toute la surface discale de la dite face avant. La base (10a) définit également un plan d'appui (10f) horizontal qui sera susceptible d'être en contact avec le clapet anti-retour (12). Le second constituant (11) comprend une base centrale (11a) circulaire se prolongeant d'un côté par une portée cylindrique (11b) susceptible de venir entourer la portée cylindrique (10c) du constituant (10) et de l'autre côté par une portée cylindrique (11c) de plus grande longueur formant manchon de protection. La base centrale (11a) se prolonge intérieurement avec une forme en saillie (11d) débordante vers le haut et susceptible d'être en contact avec le clapet anti-retour (12) par sa couronne (12a). La base centrale (11a) comprend un plan d'appui (11e) horizontal contre lequel vient en appui la face avant discale (10d), avec la formation d'une rainure circulaire (11f) susceptible de recevoir la forme en saillie (10e) précitée formée sur le constituant (10). Ainsi, le constituant (10) est centré à l'intérieur du constituant (11). Le clapet anti-retour (12), comme représenté figure 8, comprend une couronne cylindrique (12a) supérieure qui est prolongée par deux lèvres (12b) orientées inclinées l'une par rapport à l'autre à partir de la dite couronne et se rejoignant en extrémité débordante. Ces lèvres laissent une ouverture (12c) en extrémité pour le passage du lait collecté. La couronne cylindrique (12a) est ainsi positionnée dans l'espace intérieur formé entre les constituants (10) et (11) comme représenté figure 6, et plus spécifiquement dans le volume défini entre leurs bases (10a) et (11a). Par ailleurs, la forme en saillie (11c) établie sur la base (11a) du second constituant vient en contact et appui sur la partie couronne du clapet anti-retour. Le dit clapet étant réalisé en un matériau élastomère, il est donc déformé sensiblement à l'endroit de pénétration de la forme en saillie (11c) précitée assurant ainsi un blocage en position.

La liaison entre les constituants (11) et (12) peut être fixe ou démontable. Il peut y avoir un emmanchement serré après mise en place du clapet anti-retour. Il peut y avoir une liaison par des configurations d'emboîtement par clipsage à l'aide de formes complémentaires mâle-femelle formées sur les parties en regard de liaison des dits constituants.

La hauteur de la portée cylindrique (11c) est plus importante que la hauteur des lèvres (12b) du clapet anti-retour de sorte à les protéger. Ainsi la manipulation du clapet anti-retour (12) s'effectue seulement et exclusivement par une manipulation du dispositif au niveau du constituant (12) et de sa portée cylindrique (11b).

Ainsi selon l'invention, et après assemblage des constituants (10) et (11) enserrant le clapet anti-retour (12), le dispositif forme un ensemble unique constituant en quelque sorte un embout qui est ensuite positionné sur le manchon (2c) récepteur prévu à l'intérieur de la base de la téterelle. Cet ensemble peut être démonté facilement pour être ensuite manipulé à des fins de nettoyage du clapet anti-retour. Il n'est pas nécessaire de démonter ce dernier par rapport aux constituants (10) et (11) du dispositif. La manipulation du dispositif se fait par l'extérieur sans contact avec le clapet anti-retour et ainsi dans des conditions d'hygiène optimisées. Le clapet anti-retour n'est pas manipulé en soi et il n'est donc pas déformé à l'usage. La hauteur de la portée cylindrique (11c) du constituant (11) est suffisamment importante pour éviter toute pénétration de doigts de l'utilisateur avec un contact sur le clapet anti-retour.

Des éléments d'étanchéité complémentaires peuvent être prévus dans le positionnement et la liaison du dispositif sur le manchon dans la base de la téterelle. Ce sont par exemple des joints toriques ou similaires.

Les constituants (10) et (11) peuvent comporter des lumières, de section inférieure au passage d'un doigt.

Le clapet anti-retour est réalisé dans un matériau élastomère et peut être stérilisé et aussi, si besoin est, les deux constituants (10) et (11).

Les constituants (10) et (11) peuvent être réalisés en tous matériaux, plastique par exemple en étant rigide, et peuvent présenter un effet visuel de transparence ou opaque. On peut aussi envisager sans sortir du cadre de l'invention que les constituants (10) et (11) puissent constituer en eux-mêmes une seule pièce par surmoulage tout en permettant la mise en place et le clipsage en position du clapet anti-retour.

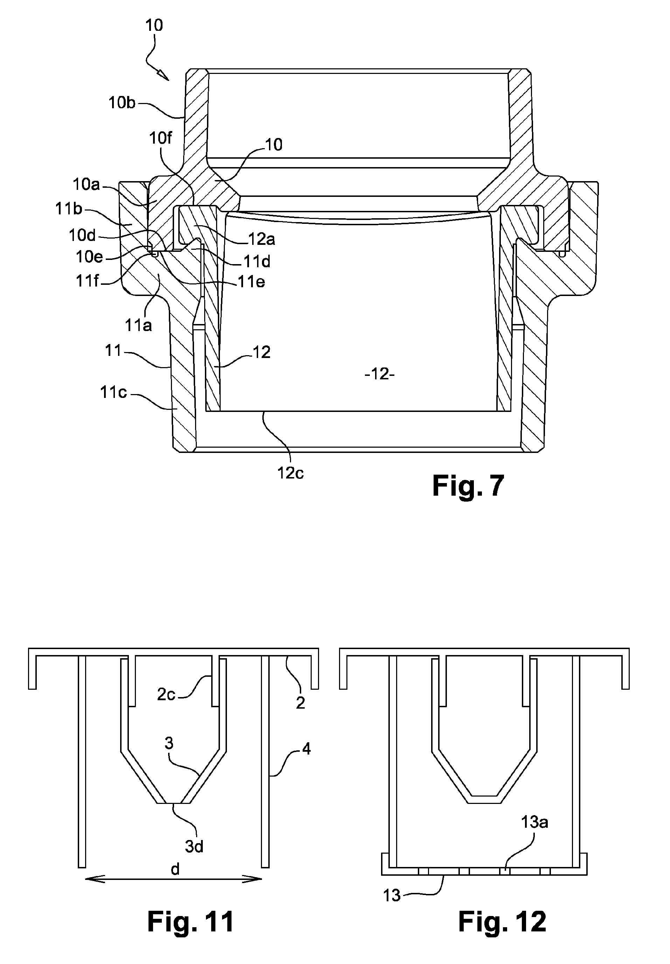

On a représenté figure 11 une autre variante de réalisation selon laquelle la téterelle est directement agencée lors de sa fabrication avec d'une part la partie manchon (2c) pour la réception du clapet anti-retour mais aussi avec le moyen (4) qui assure la protection du dit clapet anti-retour en étant d'une hauteur suffisante pour déborder de l'extrémité du dit clapet.

On a représenté figure 12 une variante d'exécution de clapet anti-retour. Celui-ci est susceptible de recevoir en extrémité inférieure, directement ou d'une manière rapportée un moyen (13) ajouré sous forme de grille, par exemple, de façon à empêcher la pénétration d'un doigt. Ce moyen est rapporté, clipsé ou autrement sur ladite extrémité du clapet anti-retour, tout en permettant l'écoulement du lait à travers les ouvertures (13a).

Sans sortir du cadre de l'invention, il reste possible pour l'utilisateur d'assurer une manipulation volontaire du clapet anti-retour mais essentiellement dans le but de son nettoyage ou de son remplacement. Dans ce cas, la hauteur du moyen support (4) ou du constituant(11) est telle qu'elle permet l'accessibilité limitée de l'utilisateur à la partie d'extrémité des lèvres du clapet anti-retour à des fins de saisie.

## Revendications

1. Dispositif anti-retour de fluides incorporé dans un ensemble d'expression de lait maternel comprenant un biberon (1) et une téterelle (2) présentant en une partie d'extrémité un bout de sein, la dite téterelle étant agencée pour la réception d'un clapet anti-retour, **caractérisé en ce que** la dite téterelle est agencée pour la réception d'un clapet anti-retour, et d'un moyen support (4) tubulaire entourant ledit clapet anti-retour (3) et débordant par rapport à l'extrémité dudit clapet anti-retour de manière à assurer sa protection totale et le protéger de tout contact involontaire des doigts de l'utilisateur.

2. Dispositif anti-retour de fluides incorporé dans un ensemble d'expression de lait maternel selon la revendication 1, la dite téterelle (2) étant agencée dans le volume intérieur de sa base avec un manchon cylindrique (2c), **caractérisé en ce que** le moyen de protection (4) forme support et est agencé intérieurement pour recevoir et maintenir un clapet anti-retour (3), le dit moyen support étant susceptible d'être positionné par rapport à la partie manchon formée dans la partie intérieure de la base (2a) constitutive du bout de sein, le dit moyen support étant de grande hauteur et assurant la protection du clapet anti-retour avec une hauteur de débordement par rapport à l'extrémité inférieure du clapet anti-retour comprise entre un millimètre et plusieurs centimètres afin d'empêcher le contact avec les doigts de l'utilisateur, et constituant le moyen de préhension et de manipulation par l'utilisateur et débouchant dans le dit biberon.

3. Dispositif anti-retour selon la revendication 2, **caractérisé en ce que** le moyen support (4) présente intérieurement une collerette (4a) configurée en forme de 'L' avec une portée cylindrique (4b) sur laquelle vient se positionner une couronne (3a) cylindrique formée sur la base du clapet anti-retour (3), **et en ce que** le dit clapet présente des lèvres (3b) qui sont convergentes pour définir un espace d'ouverture (3c) du passage du lait, **et en ce que** le moyen support est monobloc et est obtenu par moulage, **et en ce que** dans sa partie supérieure, il vient se positionner sur la partie manchon (2c) formée sur le bout de sein.

4. Dispositif anti-retour, selon la revendication 2, de fluides incorporé dans un ensemble d'expression de lait maternel comprenant un biberon (1) et une téterelle (2) présentant en une partie d'extrémité un bout de sein, la dite téterelle étant agencée dans le volume intérieur de sa base (2a) avec un manchon cylindrique (2c), **caractérisé en ce que** le dit moyen support du clapet anti-retour comprend deux constituants (10) et (11) assemblés entre eux et enserrant un clapet anti-retour (12), le premier constituant (10) étant positionné sur le dit manchon (2c), et l'autre constituant (11) assurant une double fonction d'une part de protection du clapet anti-retour (12) et d'autre part constituant le moyen exclusif de préhension et de manipulation par l'utilisateur, le dit constituant (11) débouchant dans le biberon.

5. Dispositif anti-retour selon la revendication 4, **caractérisé en ce que** le clapet anti-retour (12) comprend une couronne cylindrique (12a) supérieure prolongée par deux lèvres (12b) orientées inclinées l'une par rapport à l'autre à partir de la dite couronne et se rejoignant en extrémité débordante en délimitant une ouverture (12c) en extrémité pour le passage du lait collecté.

6. Dispositif selon les revendications 4 et 5 ensemble, **caractérisé en ce que** les constituants (10) et (11) sont agencés et assemblés en définissant un espace intérieur autorisant le positionnement de la couronne cylindrique du clapet anti-retour et en assurant le serrage et le maintien du dit clapet après assemblage, les lèvres (12b) du dit clapet se prolongeant à l'intérieur du volume définit par le constituant (11).

7. Dispositif selon la revendication 6, **caractérisé en ce que** le premier constituant (10) comprend une base centrale (10a) de configuration circulaire prolongée d'un côté par une portée cylindrique (10b) venant s'emmancher sur le manchon (2c) formé dans la base de la téterelle, et par une portée cylindrique (10c) de plus grand diamètre, **et en ce que** la dite portée (10c) présente en bout sur sa face avant (10d) un prolongement en saillie établi sur toute la surface discale de la face avant, **et en ce que** la base présente un plan d'appui (10f) horizontal constituant le contact avec le clapet anti-retour (12) par sa couronne (12a).

8. Dispositif selon la revendication 7, **caractérisé en ce que** le second constituant (11) comprend une base centrale (11a) circulaire se prolongeant d'un côté par une portée cylindrique (11b) entourant la portée cylindrique (10c) du constituant (10), et de l'autre côté par une portée cylindrique (11c) de grande longueur formant manchon de protection du clapet anti-retour, **et en ce que** la base centrale comprend une forme en saillie (11d) débordante vers le haut pour être en contact avec le clapet anti-retour par sa couronne (12a).

9. Dispositif selon la revendication 8, **caractérisé en ce que** la base centrale (11a) comprend un plan d'appui (11e) contre lequel vient en appui la face discale (10d) du constituant (10) avec la formation d'une rainure (11f) circulaire recevant la forme en saillie (10e) établie sur le constituant (10).

10. Dispositif selon la revendication 4, **caractérisé en ce que** les constituants (10) et (11) forment une seule pièce et sont agencés pour assurer la mise en place et le clipsage en position du clapet anti-retour.

11. Dispositif selon la revendication 1, **caractérisé en ce que** la téterelle est agencée directement par moulage avec le moyen de protection du clapet anti-retour.

12. Dispositif selon la revendication 2, **caractérisé en ce que** le moyen support (4) présente un diamètre d'ouverture à son extrémité inférieure comprise entre 0,5 et 3 centimètres.

13. Dispositif selon la revendication 2, **caractérisé en ce que** le moyen support (4) reçoit à son extrémité inférieure un moyen ajouré (13) formant grille.
